# EUROPÄISCHE PATENTSCHRIFT

(11) **EP 0 683 776 B1**
(45) Veröffentlichungstag und Bekanntmachung des Hinweises auf die Patenterteilung: **07.04.1999**
(21) Anmeldenummer: 94907547.7
(22) Anmeldetag: 11.02.1994
(51) Int. Cl.: C07D 405/14, C07D 417/14, C07D 409/14, A61K 31/435, C07D 401/14, C07D 413/14

(54) **SUBSTITUIERTE HETEROARYLALKYLTHIOPYRIDINE ZUR BEKÄMPFUNG VON HELICOBACTER BAKTERIEN**
SUBSTITUTED HETEROARYLALKYLTHIOPYRIDINES FOR CONTROLLING HELICOBACTER BACTERIA
HETEROARYLALKYLTHIOPYRIDINES SUBSTITUEES POUR LA LUTTE CONTRE LES BACTERIES HELICOBACTER

(30) Priorität: 17.02.1993 CH 507/93
(43) Veröffentlichungstag der Anmeldung: 29.11.1995
(73) Patentinhaber: Byk Gulden Lomberg Chemische Fabrik GmbH, 78467 Konstanz (DE)
(72) Erfinder: KOHL, Bernhard, D-78465 Konstanz (DE); SENN-BILFINGER, Jörg, D-78464 Konstanz (DE); GRUNDLER, Gerhard, D-78464 Konstanz (DE); OPFERKUCH, Wolfgang, D-44801 Bochum (DE)
(86) Internationale Anmeldenummer: EP9400393
(87) Internationale Veröffentlichungsnummer: WO9419346

(56) Entgegenhaltungen:
- WO-A-92/04898

## Beschreibung

### Anwendungsgebiet der Erfindung

Die Erfindung betrifft Verbindungen, die in der pharmazeutischen Industrie als Wirkstoffe für die Herstellung von Arzneimitteln verwendet werden sollen.

### Bekannter technischer Hintergrund

In der europäischen Patentanmeldung 150 586 werden 2-(Pyridylmethylthio- bzw. -sulfinyl)-benzimidazole offenbart, die im Pyridinteil des Moleküls in 4-Position unter anderem durch Alkylthio- oder Arylthioreste substituiert sein können. Für die beschriebenen Verbindungen wird eine langanhaltende Magensäuresekretionshemmung angegeben. - In der internationalen Patentanmeldung W089/03830 ist beschrieben, daß sich dieselben, sowie weitere strukturähnliche Verbindungen zur Behandlung der Osteoporose eignen sollen. - In der internationalen Patentanmeldung W092/12976 werden auf bestimmte Weise substituierte 2-(Pyridylmethylthio- bzw. -sulfinyl)-benzimidazole beschrieben, die gegen Helicobacter-Bakterien wirksam sein sollen und für die weiterhin offenbart ist, daß sie für die Verhütung und Behandlung einer ganzen Reihe von Erkrankungen des Magens geeignet sein sollen.

In der internationalen Patentanmeldung WO92/04898 wird die Verwendung von auf bestimmte Weise substituierten 2-(Pyridyl-methylthio- bzw. -sulfinyl)-benzimidazolen zur Herstellung von Arzneimitteln für die Bekämpfung von Helicobacter-Bakterien beansprucht.

### Beschreibung der Erfindung

Gegenstand der Erfindung sind Verbindungen der Formel I (siehe beigefügtes Formelblatt), worin
- R1: Wasserstoff, 1-4C-Alkyl oder 1-4C-Alkoxy bedeutet,
- R2: Wasserstoff, 1-4C-Alkyl, 1-4C-Alkoxy, Halogen, Trifluormethyl, ganz oder überwiegend durch Fluor substituiertes 1-4C-Alkoxy, Chlordifluormethoxy, 2-Chlor-1,1,2-trifluorethoxy oder gemeinsam mit R3 gewünschtenfalls ganz oder teilweise durch Fluor substituiertes 1-2C-Alkylendioxy oder Chlortrifluorethylendioxy bedeutet,
- R3: Wasserstoff, ganz oder überwiegend durch Fluor substituiertes 1-4C-Alkoxy, Chlordifluormethoxy, 2-Chlor-1,1,2-trifluorethoxy oder gemeinsam mit R2 gewünschtenfalls ganz oder teilweise durch Fluor substituiertes 1-2C-Alkylendioxy oder Chlortrifluorethylendioxy bedeutet,
- R4: Wasserstoff oder 1-4C-Alkyl bedeutet,
- R5: Wasserstoff, 1-4C-Alkyl oder 1-4C-Alkoxy bedeutet,
- R6: einen durch R8 und R9 substituierten Heterocyclus bedeutet, der ausgewählt ist aus der Gruppe bestehend aus Furan, Thiophen, Pyrrol, Oxazol, Isoxazol, Thiazol, Isothiazol, Imidazol, Pyrazol, 1,2,3-Triazol, 1,2,4-Triazol, Tetrazol, 1,3,4-Thiadiazol, 1,2,4-Thiadiazol, Pyrimidin und Pyridin,
- R7: Wasserstoff, 1-4C-Alkyl oder 1-4C-Alkoxy bedeutet,
- R8: Wasserstoff, 1-4C-Alkyl, 1-4C-Alkoxy, Halogen, durch R10 substituiertes 1-4C-Alkyl oder -N(R11)R12 bedeutet,
- R9: Wasserstoff, 1-4C-Alkyl, 1-4C-Alkoxy oder Halogen bedeutet,
- R10: Hydroxy, 1-4C-Alkoxy oder -N(R11)R12 bedeutet, wobei
- R11: Wasserstoff, 1-4C-Alkyl oder -CO-R13 und
- R12: Wasserstoff oder 1-4C-Alkyl bedeutet,
- R13: Wasserstoff, 1-4C-Alkyl oder 1-4C-Alkoxy bedeutet,
- m: eine Zahl von 1 bis 7 bedeutet,
- n: die Zahl 0 oder 1 bedeutet und
- p: die Zahl 0 oder 1 bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

Halogen im Sinne der vorliegenden Erfindung ist Brom, Chlor und Fluor.

Als ganz oder überwiegend durch Fluor substituiertes 1-4C-Alkoxy seien beispielsweise der 1,2,2-Trifluorethoxy, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als gewünschtenfalls ganz oder teilweise durch Fluor substituiertes 1-2C-Alkylendioxy seien beispielsweise der Methylendioxy- (-O-CH₂-O-), der Ethylendioxy- (-O-CH₂-CH₂-O-), der 1,1-Difluorethylendioxy- (-O-CF₂-CH₂-O-), der 1,1,2,2-Tetrafluorethylendioxy- (-O-CF₂-CF₂-O-) und insbesondere der Difluormethylendioxy- (-O-CF₂-O-) und der 1,1,2-Trifluorethylendioxyrest (-O-CF₂-CHF-O-) genannt.

Wenn R2 und R3 gemeinsam gewünschtenfalls ganz oder teilweise durch Fluor substituiertes 1-2C-Alkylendioxy oder Chlortrifluorethylendioxy bedeuten, so sind die Substituenten R2 und R3 in Nachbarpositionen - bevorzugt an den Positionen 5 und 6 - am Benzoteil des Benzimidazolringes gebunden.

Die Heterocyclen R6 können über jede denkbare Position an den Rest -CₘH₂ₘ- angebunden sein, so daß als Reste R6 beispielsweise genannt seien die Reste: 2-Furyl, 3-Furyl, 2-Thienyl, 3-Thienyl, 1-Pyrrolyl, 2-Pyrrolyl, 3-Pyrrolyl, 2-Oxazolyl, 4-Oxazolyl, 5-Oxazolyl, 3-Isoxazolyl, 4-Isoxazolyl, 5-Isoxazolyl, 2-Thiazolyl, 4-Thiazolyl, 5-Thiazolyl, 3-Isothiazolyl, 4-Isothiazolyl, 5-Isothiazolyl, 1-Imidazolyl, 2-Imidazolyl, 4-Imidazolyl, 1-Pyrazolyl, 3-Pyrazolyl, 4-Pyrazolyl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-1-yl, Tetrazol-2-yl, Tetrazol-5-yl, 1,3,4-Thiadiazol-2-yl, 1,2,4-Thiadiazol-3-yl, 2-Pyridinyl, 4-Pyridinyl und 2-Pyrimidinyl.

Die Substituenten R8 und gegebenenfalls R9 können in den Heterocyclen R6 an jeder denkbaren Position angebunden sein. Als beispielhafte substituierte Heterocyclen R6 seien genannt die Reste: 3-Methyl-2-furyl, 2-Methyl-3-furyl, 5-Methyl-2-furyl, 5-Ethyl-2-furyl, 2-Dimethylaminomethyl-5-methyl-3-furyl, 3-Methoxy-2-furyl, 5-Dimethylaminomethyl-2-furyl, 5-Methoxymethyl-2-furyl, 5-Hydroxymethyl-2-furyl, 5-Chlor-2-furyl, 5-Fluor-2-furyl, 5-Methyl-2-thienyl, 5-Chlor-2-thienyl, 3-Methyl-2-thienyl, 3-Amino-2-thienyl, 3-Methoxy-2-thienyl, 2-Methyl-3-thienyl, 5-Dimethylaminomethyl-2-thienyl, 5-Methyl-2-pyrrolyl, 2,5-Dimethyl-1-pyrrolyl, 1,5-Dimethyl-2-pyrrolyl, 1-Methyl-2-pyrrolyl, 2-Amino-4-thiazolyl, 2-Methyl-4-thiazolyl, 2-Amino-5-methyl-4-thiazolyl, 4-Methyl-5-thiazolyl, 2-Dimethylaminomethyl-4-thiazolyl, 4,5-Dimethyl-2-thiazolyl, 2-Formylamino-4-thiazolyl, 4-Methyl-5-oxazolyl, 2-Methyl-4-imidazolyl, 5-Methyl-4-imidazolyl, 2-Methyl-1-imidazolyl, 4,5-Dimethyl-2-imidazolyl, 4-Hydroxymethyl-5-methyl-1-imidazolyl, 3-Methyl-1-pyrazolyl, 5-Amino-1,2,4-thiadiazol-3-yl, 4-Methoxy-2-pyridinyl, 4-Methoxy-3-methyl-2-pyridinyl und 3,4-Dimethoxypyridinyl.

Als Reste -CₘH_{2m,} die durch R6 substituiert sind, kommen geradkettige oder verzweigte Reste infrage. Beispielsweise seien genannt der Heptyl-, Isoheptyl- (2-Methylhexyl-), Hexyl-, Isohexyl- (2-Methylpentyl-), Neohexyl-(2,2-Dimethylbutyl-), Pentyl-, Isopentyl- (3-Methylbutyl-), Neopentyl(2,2-Dimethylpropyl-), Butyl-, iso-Butyl-, sec.-Butyl-, tert.-Butyl-, Propyl-, Isopropyl-, Ethyl- und der Methylrest. Als beispielhafte Reste -CₘH₂ₘ-R6 seien genannt die Reste: 3-Methyl-2-furyl-methyl, 3-Methyl-2-furyl-ethyl, 2-Furyl-methyl, 2-Furyl-ethyl, 2-Furyl-propyl, 2-Furyl-butyl, 5-Dimethylaminomethyl-2-furyl-methyl, 5-Dimethylaminomethyl-2-furyl-ethyl, 5-Dimethylaminomethyl-2-furyl-propyl, 2-Methyl-3-furyl-methyl, 2-Methyl-3-furyl-ethyl, 3-Methoxy-2-furyl-methyl, 3-Methoxy-2-furyl-ethyl, 3-Amino-2-thienyl-methyl, 3-Amino-2-thienyl-ethyl, 5-Dimethylaminomethyl-2-thienyl-methyl, 1-Methyl-2-pyrrolyl-methyl, 2-Amino-4-thiazolyl-methyl, 2-Dimethylaminomethyl-4-thiazolyl-methyl, 5-Methyl-4-imidazolyl-methyl, 4-Hydroxymethyl-5-methyl-1-imidazolyl-methyl, 5-Dimethylaminomethyl-2-thienyl-ethyl, 1-Methyl-2-pyrrolyl-ethyl, 2-Amino-4-thiazolyl-ethyl, 2-Dimethylaminomethyl-4-thiazolyl-ethyl, 5-Methyl-4-imidazolyl-ethyl, 4-Hydroxymethyl-5-methyl-1-imidazolyl-ethyl, 5-Amino-(1,2,4-thiadiazol-3-yl)-methyl und 5-Amino-(1,2,4-thiadiazol-3-yl)-ethyl.

Als Salze kommen für Verbindungen der Formel I, in denen n die Zahl 0 bedeutet, alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Für Verbindungen der Formel I, in denen n die Zahl 1 bedeutet, kommen als Salze auch Salze mit Basen in Betracht. Als Beispiele für basische Salze seien Lithium-, Natrium-, Kalium-, Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Besonders erwähnenswerte Verbindungen sind in den Unteransprüchen genannt.

Hervorzuhebende Verbindungen sind solche der Formel I, worin
- R1: Wasserstoff bedeutet,
- R2: Wasserstoff, 1-4C-Alkoxy, Trifluormethyl, ganz oder überwiegend durch Fluor substituiertes 1-4C-Alkoxy, oder gemeinsam mit R3 ganz oder teilweise durch Fluor substituiertes 1-2C-Alkylendioxy bedeutet,
- R3: Wasserstoff oder gemeinsam mit R2 ganz oder teilweise durch Fluor substituiertes 1-2C-Alkylendioxy bedeutet,
- R4: Wasserstoff bedeutet,
- R5: Wasserstoff, 1-4C-Alkyl oder 1-4C-Alkoxy bedeutet,
- R6: einen durch R8 und R9 substituierten Heterocyclus bedeutet, der ausgewählt ist aus der Gruppe bestehend aus Furan, Thiophen, Thiazol, Imidazol und Pyridin,
- R7: Wasserstoff bedeutet,
- R8: Wasserstoff, 1-4C-Alkyl, 1-4C-Alkoxy oder durch R10 substituiertes 1-4C-Alkyl bedeutet,
- R9: Wasserstoff oder 1-4C-Alkoxy bedeutet,
- R10: -N(R11)R12 bedeutet, wobei
- R11: 1-4C-Alkyl und
- R12: 1-4C-Alkyl bedeutet,
- m: eine Zahl von 1 bis 3 bedeutet,
- n: die Zahl 0 oder 1 bedeutet und
- p: die Zahl 0 bedeutet,
und ihre Salze.

Besonders hervorzuhebende Verbindungen sind solche der Formel I, worin
- R1: Wasserstoff bedeutet,
- R2: Wasserstoff oder Methoxy bedeutet,
- R3: Wasserstoff bedeutet,
- R4: Wasserstoff bedeutet,
- R5: Wasserstoff, 1-4C-Alkyl oder 1-4C-Alkoxy bedeutet,
- R6: einen durch R8 und R9 substituierten Heterocyclus bedeutet, der ausgewählt ist aus der Gruppe bestehend aus Furan, Thiophen, Thiazol, Imidazol und Pyridin,
- R7: Wasserstoff bedeutet,
- R8: Wasserstoff, 1-4C-Alkyl, 1-4C-Alkoxy oder durch R10 substituiertes 1-4C-Alkyl bedeutet,
- R9: Wasserstoff oder 1-4C-Alkoxy bedeutet,
- R10: -N(R11)R12 bedeutet, wobei
- R11: 1-4C-Alkyl und
- R12: 1-4C-Alkyl bedeutet,
- m: eine Zahl von 1 bis 3 bedeutet,
- n: die Zahl 0 bedeutet und
- p: die Zahl 0 bedeutet,
und ihre Salze.

Beispielhafte erfindungsgemäße Verbindungen sind in der folgenden Tabelle 1 aufgeführt:

**TABELLE 1**

| Verbindungen der Formel I (siehe beigefügtes Formelblatt) mit den folgenden Substituentenbedeutungen: | | | | | | | | | |
|---|---|---|---|---|---|---|---|---|---|
| R1 | R2 | R3 | R4 | R5 | R6 | R7 | m | n | p |
| H | H | H | H | H | 2-Furyl | H | 1 | 0 | 0 |
| H | H | H | H | H | 2-Thienyl | H | 1 | 0 | 0 |
| H | H | H | H | H | 3-Thienyl | H | 1 | 0 | 0 |
| H | H | H | H | H | 5-Dimethylaminomethyl-2-furyl | H | 1 | 0 | 0 |
| H | H | H | H | H | 5-Piperidinomethyl-2-furyl | H | 1 | 0 | 0 |
| H | H | H | H | H | 5-Dimethylaminomethyl-2-thienyl | H | 1 | 0 | 0 |
| H | H | H | H | H | 2-Amino-4-thiazolyl | H | 1 | 0 | 0 |
| H | H | H | H | H | 2-Dimethylaminomethyl-4-thiazolyl | H | 1 | 0 | 0 |
| H | H | H | H | H | 2-Guanidino-4-thiazolyl | H | 1 | 0 | 0 |
| H | H | H | H | H | 2-Formylamino-4-thiazolyl | H | 1 | 0 | 0 |
| H | H | H | H | H | 4-Methyl-5-oxazolyl | H | 1 | 0 | 0 |
| H | H | H | H | H | 2-Methyl-4-imidazolyl | H | 1 | 0 | 0 |
| H | H | H | H | H | 5-Methyl-4-imidazolyl | H | 1 | 0 | 0 |
| H | H | H | H | H | 5-Amino-1,2,4-thiadiazol-3-yl | H | 1 | 0 | 0 |
| H | F | H | H | H | 2-Furyl | H | 1 | 0 | 0 |
| H | OCH₃ | H | H | H | 2-Furyl | H | 1 | 0 | 0 |
| H | OCH₃ | H | H | H | 2-Thienyl | H | 1 | 0 | 0 |
| H | OCH₃ | H | H | H' | 5-Dimethylaminomethyl-2-furyl | H | 1 | 0 | 0 |
| H | OCH₃ | H | H | H | 5-Dimethylaminomethyl-2-thienyl | H | 1 | 0 | 0 |
| H | OCH₃ | H | H | H | 2-Amino-4-thiazolyl | H | 1 | 0 | 0 |
| H | OCH₃ | H | H | H | 2-Dimethylaminomethyl-4-thiazolyl | H | 1 | 0 | 0 |
| H | OCH₃ | H | H | H | 2-Guanidino-4-thiazolyl | H | 1 | 0 | 0 |
| H | OCH₃ | H | H | H | 2-Formylamino-4-thiazolyl | H | 1 | 0 | 0 |
| H | OCH₃ | H | H | H | 4-Methyl-5-thiazolyl | H | 1 | 0 | 0 |
| H | OCH₃ | H | H | H | 4-Methyl-5-oxazolyl | H | 1 | 0 | 0 |
| H | OCH₃ | H | H | H | 2-Methyl-4-imidazolyl | H | 1 | 0 | 0 |
| H | OCH₃ | H | H | H | 5-Methyl-4-imidazolyl | H | 1 | 0 | 0 |
| H | H | H | H | CH₃ | 2-Furyl | H | 1 | 0 | 0 |
| H | H | H | H | CH₃ | 2-Thienyl | H | 1 | 0 | 0 |
| H | H | H | H | CH₃ | 5-Dimethylaminomethyl-2-furyl | H | 1 | 0 | 0 |
| H | H | H | H | CH₃ | 5-Dimethylaminomethyl-2-thienyl | H | 1 | 0 | 0 |
| H | H | H | H | CH₃ | 2-Amino-4-thiazolyl | H | 1 | 0 | 0 |
| H | H | H | H | CH₃ | 2-Dimethylaminomethyl-4-thiazolyl | H | 1 | 0 | 0 |
| H | H | H | H | CH₃ | 2-Guanidino-4-thiazolyl | H | 1 | 0 | 0 |
| H | H | H | H | CH₃ | 2-Formylamino-4-thiazolyl | H | 1 | 0 | 0 |
| H | H | H | H | CH₃ | 4-Methyl-5-oxazolyl | H | 1 | 0 | 0 |
| H | H | H | H | CH₃ | 2-Methyl-4-imidazolyl | H | 1 | 0 | 0 |
| H | H | H | H | CH₃ | 5-Methyl-4-imidazolyl | H | 1 | 0 | 0 |
| H | F | H | H | CH₃ | 2-Furyl | H | 1 | 0 | 0 |
| H | H | H | H | OCH₃ | 2-Furyl | H | 1 | 0 | 0 |
| H | H | H | H | OCH₃ | 2-Thienyl | H | 1 | 0 | 0 |
| H | H | H | H | OCH₃ | 5-Dimethylaminomethyl-2-furyl | H | 1 | 0 | 0 |
| H | H | H | H | OCH₃ | 5-Dimethylaminomethyl-2-thienyl | H | 1 | 0 | 0 |
| H | H | H | H | OCH₃ | 2-Amino-4-thiazolyl | H | 1 | 0 | 0 |
| H | H | H | H | OCH₃ | 2-Dimethylaminomethyl-4-thiazolyl | H | 1 | 0 | 0 |
| H | H | H | H | OCH₃ | 2-Guanidino-4-thiazolyl | H | 1 | 0 | 0 |
| H | H | H | H | OCH₃ | 2-Formylamino-4-thiazolyl | H | 1 | 0 | 0 |
| H | H | H | H | OCH₃ | 4-Methyl-5-oxazolyl | H | 1 | 0 | 0 |
| H | H | H | H | OCH₃ | 2-Methyl-4-imidazolyl | H | 1 | 0 | 0 |
| H | H | H | H | OCH₃ | 5-Methyl-4-imidazolyl | H | 1 | 0 | 0 |
| H | F | H | H | OCH₃ | 2-Furyl | H | 1 | 0 | 0 |
| H | H | H | H | H | 2-Furyl | H | 2 | 0 | 0 |
| H | H | H | H | H | 2-Thienyl | H | 2 | 0 | 0 |
| H | H | H | H | H | 5-Dimethylaminomethyl-2-furyl | H | 2 | 0 | 0 |
| H | H | H | H | H | 5-Dimethylaminomethyl-2-thienyl | H | 2 | 0 | 0 |
| H | H | H | H | H | 2-Amino-4-thiazolyl | H | 2 | 0 | 0 |
| H | H | H | H | H | 2-Dimethylaminomethyl-4-thiazolyl | H | 2 | 0 | 0 |
| H | H | H | H | H | 2-Guanidino-4-thiazolyl | H | 2 | 0 | 0 |
| H | H | H | H | H | 2-Formylamino-4-thiazolyl | H | 2 | 0 | 0 |
| H | H | H | H | H | 4-Methyl-5-oxazolyl | H | 2 | 0 | 0 |
| H | H | H | H | H | 2-Methyl-4-imidazolyl | H | 2 | 0 | 0 |
| H | H | H | H | H | 5-Methyl-4-imidazolyl | H | 2 | 0 | 0 |
| H | OCH₃ | H | H | H | 2-Furyl | H | 2 | 0 | 0 |
| H | OCH₃ | H | H | H | 2-Thienyl | H | 2 | 0 | 0 |
| H | OCH₃ | H | H | H | 5-Dimethylaminomethyl-2-furyl | H | 2 | 0 | 0 |
| H | OCH₃ | H | H | H | 5-Dimethylaminomethyl-2-thienyl | H | 2 | 0 | 0 |
| H | OCH₃ | H | H | H | 2-Amino-4-thiazolyl | H | 2 | 0 | 0 |
| H | OCH₃ | H | H | H | 2-Dimethylaminomethyl-4-thiazolyl | H | 2 | 0 | 0 |
| H | OCH₃ | H | H | H | 2-Guanidino-4-thiazolyl | H | 2 | 0 | 0 |
| H | OCH₃ | H | H | H | 2-Formylamino-4-thiazolyl | H | 2 | 0 | 0 |
| H | OCH₃ | H | H | H | 4-Methyl-5-oxazolyl | H | 2 | 0 | 0 |
| H | OCH₃ | H | H | H | 2-Methyl-4-imidazolyl | H | 2 | 0 | 0 |
| H | OCH₃ | H | H | H | 5-Methyl-4-imidazolyl | H | 2 | 0 | 0 |
| H | H | H | H | CH₃ | 2-Furyl | H | 2 | 0 | 0 |
| H | H | H | H | CH₃ | 2-Thienyl | H | 2 | 0 | 0 |
| H | H | H | H | CH₃ | 5-Dimethylaminomethyl-2-furyl | H | 2 | 0 | 0 |
| H | H | H | H | CH₃ | 5-Dimethylaminomethyl-2-thienyl | H | 2 | 0 | 0 |
| H | H | H | H | CH₃ | 2-Amino-4-thiazolyl | H | 2 | 0 | 0 |
| H | H | H | H | CH₃ | 2-Dimethylaminomethyl-4-thiazolyl | H | 2 | 0 | 0 |
| H | H | H | H | CH₃ | 2-Guanidino-4-thiazolyl | H | 2 | 0 | 0 |
| H | H | H | H | CH₃ | 2-Formylamino-4-thiazolyl | H | 2 | 0 | 0 |
| H | H | H | H | CH₃ | 4-Methyl-5-oxazolyl | H | 2 | 0 | 0 |
| H | H | H | H | CH₃ | 2-Methyl-4-imidazolyl | H | 2 | 0 | 0 |
| H | H | H | H | CH₃ | 5-Methyl-4-imidazolyl | H | 2 | 0 | 0 |
| H | H | H | H | OCH₃ | 2-Furyl | H | 2 | 0 | 0 |
| H | H | H | H | OCH₃ | 2-Thienyl | H | 2 | 0 | 0 |
| H | H | H | H | OCH₃ | 5-Dimethylaminomethyl-2-furyl | H | 2 | 0 | 0 |
| H | H | H | H | OCH₃ | 5-Dimethylaminomethyl-2-thienyl | H | 2 | 0 | 0 |
| H | H | H | H | OCH₃ | 2-Amino-4-thiazolyl | H | 2 | 0 | 0 |
| H | H | H | H | OCH₃ | 2-Dimethylaminomethyl-4-thiazolyl | H | 2 | 0 | 0 |
| H | H | H | H | OCH₃ | 2-Guanidino-4-thiazolyl | H | 2 | 0 | 0 |
| H | H | H | H | OCH₃ | 2-Formylamino-4-thiazolyl | H | 2 | 0 | 0 |
| H | H | H | H | OCH₃ | 4-Methyl-5-oxazolyl | H | 2 | 0 | 0 |
| H | H | H | H | OCH₃ | 2-Methyl-4-imidazolyl | H | 2 | 0 | 0 |
| H | H | H | H | OCH₃ | 5-Methyl-4-imidazolyl | H | 2 | 0 | 0 |
| H | OCH₃ | H | H | H | 3-Thienyl | H | 1 | 0 | 0 |
| H | H | H | H | CH₃ | 3-Thienyl | H | 1 | 0 | 0 |
| H | H | H | H | OCH₃ | 3-Thienyl | H | 1 | 0 | 0 |
| H | H | H | H | H | 3-Thienyl | H | 2 | 0 | 0 |
| H | OCH₃ | H | H | H | 3-Thienyl | H | 2 | 0 | 0 |
| H | H | H | H | CH₃ | 3-Thienyl | H | 2 | 0 | 0 |
| H | H | H | H | OCH₃ | 3-Thienyl | H | 2 | 0 | 0 |
| H | OCH₃ | H H | H | H | 5-Piperidinomethyl-2-furyl | H | 1 | 0 | 0 |
| H | H | H | H | CH₃ | 5-Piperidinomethyl-2-furyl | H | 1 | 0 | 0 |
| H | H | H | H | OCH₃ | 5-Piperidinomethyl-2-furyl | H | 1 | 0 | 0 |
| H | H | H | H | H | 5-Piperidinomethyl-2-furyl | H | 2 | 0 | 0 |
| H | OCH₃ | H | H | H | 5-Piperidinomethyl-2-furyl | H | 2 | 0 | 0 |
| H | H | H | H | CH₃ | 5-Piperidinomethyl-2-furyl | H | 2 | 0 | 0 |
| H | H | H | H | OCH₃ | 5-Piperidinomethyl-2-furyl | H | 2 | 0 | 0 |
| H | OCH₃ | H | H | H | 5-Amino-1,2,4-thiadiazol-3-yl | H | 1 | 0 | 0 |
| H | H | H | H | CH₃ | 5-Amino-1,2,4-thiadiazol-3-yl | H | 1 | 0 | 0 |
| H | H | H | H | OCH₃ | 5-Amino-1,2,4-thiadiazol-3-yl | H | 1 | 0 | 0 |
| H | H | H | H | H | 5-Amino-1,2,4-thiadiazol-3-yl | H | 2 | 0 | 0 |
| H | OCH₃ | H | H | H | 5-Amino-1,2,4-thiadiazol-3-yl | H | 2 | 0 | 0 |
| H | H | H | H | CH₃ | 5-Amino-1,2,4-thiadiazol-3-yl | H | 2 | 0 | 0 |
| H | H | H | H | OCH₃ | 5-Amino-1,2,4-thiadiazol-3-yl | H | 2 | 0 | 0 |
| H | H | H | H | H | 4-Methyl-5-thiazolyl | H | 1 | 0 | 0 |
| H | H | H | H | CH₃ | 4-Methyl-5-thiazolyl | H | 1 | 0 | 0 |
| H | H | H | H | OCH₃ | 4-Methyl-5-thiazolyl | H | 1 | 0 | 0 |
| H | H | H | H | H | 4-Methyl-5-thiazolyl | H | 2 | 0 | 0 |
| H | OCH₃ | H | H | H | 4-Methyl-5-thiazolyl | H | 2 | 0 | 0 |
| H | H | H | H | CH₃ | 4-Methyl-5-thiazolyl | H | 2 | 0 | 0 |
| H | H | H | H | OCH₃ | 4-Methyl-5-thiazolyl | H | 2 | 0 | 0 |

und die Salze dieser Verbindungen.

Ein weiterer Gegenstand der Erfindung ist ein Verfahren zur Herstellung der Verbindungen der Formel I, worin R1, R2, R3, R4, R5, R6, R7, m, n und p die oben angegebenen Bedeutungen haben, und ihrer Salze.

Das Verfahren ist dadurch gekennzeichnet, daß man Mercaptobenzimidazole der Formel II (siehe beigefügtes Formelblatt), worin R1, R2, R3 und R4 die oben angegebenen Bedeutungen haben, mit Picolinderivaten III (siehe beigefügtes Formelblatt), worin R5, R6, R7, m und p die oben angegebenen Bedeutungen haben und X eine geeignete Abgangsgruppe darstellt, umsetzt und (falls Verbindungen der Formel I mit n=1 die gewünschten Endprodukte sind) anschliessend die erhaltenen 2-Benzimidazolyl-2-pyridylmethyl-sulfide der Formel I mit n=O oxydiert und/oder gewünschtenfalls in die Salze überführt.

Bei der vorstehend aufgeführten Umsetzung können die Verbindungen II und III als solche oder gegebenenfalls in Form ihrer Salze eingesetzt werden.

Die Umsetzung von II mit III erfolgt in geeigneten, vorzugsweise polaren protischen oder aprotischen Lösungsmitteln (wie Methanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin.

Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen 0° und 150°C liegen, wobei in Gegenwart von Protonenakzeptoren Temperaturen zwischen 20° und 80°C und ohne Protonenakzeptoren zwischen 60° und 120°C - insbesondere die Siedetemperatur der verwendeten Lösungsmittel - bevorzugt sind. Die Reaktionszeiten liegen zwischen 0,5 und 12 Stunden.

Die Oxidation der Sulfide (Verbindungen der Formel I mit n=0) zu den Sulfoxiden (Verbindungen der Formel I mit n=1) erfolgt unter den Bedingungen, wie sie dem Fachmann für die Oxidation von Sulfiden zu Sulfoxiden geläufig sind [siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980]. Als Oxidationsmittel kommen alle für die Oxidation von Sulfiden zu Sulfoxiden üblicherweise verwendeten Reagenzien in Frage, insbesondere Peroxysäuren, wie z.B. Peroxyessigsäure, Trifluorperoxyessigsäure, 3,5-Dinitroperoxybenzoesäure, Peroxymaleinsäure, Magnesiummonoperoxiphthalat oder bevorzugt m-Chlorperoxybenzoesäure.

Die Reaktionstemperatur liegt (je nach Reaktivität des Oxidationsmittels und Verdünnungsgrad) zwischen -70°C und der Siedetemperatur des verwendeten Lösungsmittels, bevorzugt jedoch zwischen -30° und +20°C. Als vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen 0° und 50°C durchgeführt wird. Die Reaktion wird zweckmäßigerweise in inerten Lösungsmitteln, z.B. aromatischen oder chlorierten Kohlenwasserstoffen, wie Benzol, Toluol, Dichlormethan oder Chloroform, vorzugsweise in Estern oder Ethern, wie Essigsäureethylester, Essigsäureisopropylester oder Dioxan, oder in Alkoholen, vorzugsweise Isopropanol, durchgeführt.

Die erfindungsgemäßen Sulfoxide sind optisch aktive Verbindungen. Je nach Art der Substituenten können noch weitere Chiralitätszentren im Molekül sein. Die Erfindung umfaßt daher sowohl die Enantiomeren und Diastereomeren als auch ihre Mischungen und Racemate. Die Enantiomeren können in an sich bekannter Weise (beispielsweise durch Herstellung und Trennung entsprechender diastereoisomerer Verbindungen) separiert werden (siehe z.B. WO92/08716).

Die Verbindungen II sind z.B. aus W086/02646, EP 134 400 oder EP 127 763 bekannt. Die Verbindungen III mit p=0 können beispielsweise so wie in den nachfolgenden Beispielen beschrieben hergestellt werden.

Für Verbindungen III mit p=1 werden die entsprechenden 2-Hydroxymethyl-4-mercaptosubstituierten Pyridine beispielsweise mit m-Chlorperoxibenzoesäure zu den Sulfoxiden oxidiert und anschließend beispielsweise mit Thionylchlorid chloriert. Umsetzung mit 2-Mercaptobenzimidazolen liefert die Verbindungen der Formel I mit p=1.

Die zur Herstellung von III benötigten Thiole R6-CₘH₂ₘ-SH können z.B. aus den entsprechenden Halogenverbindungen analog J. Med. Chem. 14 (1971) 349 hergestellt werden.

Die folgenden Beispiele dienen der näheren Erläuterung zur Herstellung der erfindungsgemäßen Verbindungen. Insbesondere dienen die Beispiele auch dazu, Herstellung ausgewählter Ausgangsverbindungen exemplarisch zu beschreiben. Ebenso können weitere Verbindungen der Formel I sowie weitere Ausgangsverbindungen, deren Herstellung nicht explizit beschrieben ist, in analoger oder in einer dem Fachmann an sich vertrauten Weise unter Anwendung üblicher Verfahrenstechniken hergestellt werden. Die Abkürzung RT steht für Raumtemperatur, h steht für Stunde(n), Schmp. für Schmelzpunkt, Zers. für Zersetzung.

### Beispiele

### Endprodukte

### 1. 2-{[[4-(2-Furylmethylthio)-3-methyl-2-pyridinyl]methyl]thio}-1H-benzimidazol

Zu einer Lösung von 2-Mercapto-1H-benzimidazol (1,5 g/10 mMol) in 40 ml Ethanol und 21 ml 1 n Natronlauge wird ein Äquivalent (2,92 g) 2-Chlormethyl-4-(2-furylmethylthio)-3-methylpyridin-hydrochlorid (gelöst in 10 ml Wasser) innerhalb von 20 Min. bei 40°C zugetropft. Man rührt anschließend 2 - 3 h bei 50-60°C und weitere 3 - 4 h bei RT, destilliert Ethanol am Rotationsverdampfer (1 kPa/40°C) ab, extrahiert 3 mal mit je 20 ml Dichlormethan, wäscht mit 0,1 n Natronlauge, trocknet über Kaliumcarbonat und engt im Vakuum vollständig ein. Zur Reinigung wird das Rohprodukt an Kieselgel chromatographiert (Dichlormethan/Methanol 20:1 bis 3:1); die gesammelten reinen Fraktionen werden gemeinsam im Vakuum eingeengt und aus Dichlormethan/Diisopropylether zur Kristallisation gebracht. Anschließend wird aus Methanol/Toluol umkristallisiert. Ausbeute: 2,61 g (71 %) der Titelverbindung als farbloser Feststoff vom Schmp. 188-189°C.

### 2. 2-{[[4-(2-Furylmethylthio)-3-methoxy-2-pyridinyl]methyl]thio}-1H-benzimidazol

Nach der in Beispiel 1 angegebenen Arbeitsweise erhält man ausgehend von den Ausgangsverbindungen 2-Mercapto-1H-benzimidazol und 2-Chlormethyl-4-(2-furylmethylthio)-3-methoxypyridin-hydrochlorid nach Chromatographie an Kieselgel und anschließender Kristallisation aus Ethanol/Wasser die Titelverbindung als farbloses Pulver vom Schmp. 102-103°C (Ausbeute 68 %).

### 3. 2-{[[3-Methyl-4-[2-(4-methyl-5-thiazolyl)-ethylthio]-2-pyridinyl]methyl]thio}-1H-benzimidazol

Nach der im Beispiel 1 angegebenen Arbeitsweise erhält man durch Umsetzung von 2-Mercapto-1H-benzimidazol mit 2-Chlormethyl-3-methyl-4-[2-(4-methyl-5-thiazolyl)-ethylthio]pyridin-hydrochlorid nach Kristallisation aus Dichlormethan/Diisopropylether die Titelverbindung als farblosen Feststoff vom Schmp. 150-152°C.

### 4. 2-{[[4-[(5-Dimethylaminomethyl-2-furyl)methylthio]-3-methyl-2-pyridinyl]methyl]thio}-1H-benzimidazol

Nach der im Beispiel 1 angegebenen Arbeitsweise erhält man durch Umsetzung von 2-Mercapto-1H-benzimidazol mit 2-Chlormethyl-4-{[(5-dimethylaminomethyl-2-furyl)methyl]thio}-3-methylpyridin-hydrochlorid nach Chromatographie an Kieselgel (Dichlormethan/Methanol/Triethylamin 9:1:0,1 bis 2:1:0,1) die Titelverbindung.

H NMR (CDCl₃): δ 2.24 (s, 6H); 2.33 (s, 3H); 3.41 (s, 2H); 4.15 (s, 2H); 4.43 (s, 2H); 6.10-6.21 (m, 2H); 7.10-7.25 (m, 3H); 7.40-7.60 (m, 2H); 8.27 (d, J=5.4 Hz, 1H).

Durch Auflösen in Isopropanol, Zugabe von wäßriger konzentrierter Salzsäure, Einengen zur Trockene und Kristallisation des Rückstandes aus Methanol/Aceton erhält man ein Hydrochloridsalz der Titelverbindung als farbloses Kristallpulver vom Schmp. 218°C (Zers.).

Auf analoge Weise werden folgende Verbindungen hergestellt:

### 5. 2-{[[3-Methyl-4-(2-thienylmethylthio)-2-pyridinyl]methyl]thio}-1H-benzimidazol

2-Chlormethyl-3-methyl-4-(2-thienylmethylthio)-pyridin-hydrochlorid, 2-Mercapto-1H-benzimidazol und Natriumhydroxid in Ethanol werden 20 h bei 25°C umgesetzt. Nach Kristallisation aus Toluol/Methanol wird die Titelverbindung isoliert. Ausbeute: 75 %, Schmp. 131-133°C.

### 6. 2-{[[3-Methyl-4-(3-thienylmethylthio)-2-pyridinyl]methyl]thio}-1H-benzimidazol

2-Chlormethyl-3-methyl-4-(3-thienylmethylthio)-pyridin-hydrochlorid, 2-Mercapto-1H-benzimidazol und Natriumhydroxid in Ethanol werden 20 h bei 25°C umgesetzt. Nach Kristallisation aus Toluol/Methanol wird die Titelverbindung isoliert. Ausbeute: 62 %, Schmp. 161-164°C.

### 7. 2-{[[3-Methoxy-4-(2-thienylmethylthio)-2-pyridinyl]methyl]thio}-1H-benzimidazol

2-Chlormethyl-3-methoxy-4-(2-thienylmethylthio)-pyridin-hydrochlorid, 2-Mercapto-lH-benzimidazol und Natriumhydroxid in Ethanol werden 20 h bei 25°C umgesetzt. Nach Kristallisation aus Toluol/Methanol wird die Titelverbindung isoliert. Ausbeute: 56 %, Schmp. 124-127°C.

### 8. 2-{[[4-(2-Thienylmethylthio)-2-pyridinyl]methyl]thio}-1H-benzimidazol

2-Chlormethyl-4-(2-thienylmethylthio)-pyridin-hydrochlorid, 2-Mercapto-1H-benzimidazol und Natriumhydroxid in Ethanol werden 5 h bei 60°C umgesetzt. Nach Kristallisation aus Toluol/Methanol wird die Titelverbindung isoliert. Ausbeute: 69 %, Schmp. 221-222°C (Zers.).

### 9. 2-{[[4-(2-Furylmethylthio)-2-pyridinyl]methyl]thio}-1H-benzimidazol

2-Chlormethyl-4-(2-furylmethylthio)-pyridin-hydrochlorid, 2-Mercapto-1H-benzimidazol und Natriumhydroxid in Ethanol werden 5 h bei 60°C umgesetzt. Nach Kristallisation aus Toluol/Methanol wird die Titelverbindung isoliert. Ausbeute: 73 %, Schmp. 208-209°C.

### 10. 5-Methoxy-2-{[[4-(2-furylmethylthio)-3-methyl-2-pyridinyl]methyl]thio}-1H-benzimidazol

2-Chlormethyl-4-(2-furylmethylthio)-3-methylpyridin-hydrochlorid, 5-Methoxy-2-mercapto-lH-benzimidazol und Natriumhydroxid in Ethanol werden 4 h bei 60°C umgesetzt. Man erhält die Titelverbindung als farblosen Feststoff. Ausbeute: 81 %; Schmp. 106-108°C.

### 11. 5-Methoxy-2-{[[3-methyl-4-[2-(4-methyl-5-thiazolyl)-ethylthio]-2-pyridinyl]methyl]thio}-1H-benzimidazol

Nach der in Beispiel 1 angegebenen Arbeitsweise erhält man durch Umsetzung von 5-Methoxy-2-mercapto-lH-benzimidazol mit 2-Chlormethyl-3-methyl-4-[2-(4-methyl-5-thiazolyl)-ethylthio]pyridin-hydrochlorid und wäßriger Natronlauge die Titelverbindung als gelbes Öl, aus dem nach der in Beispiel 4 angegebenen Arbeitsweise ein Hydrochloridsalz der Titelverbindung als farbloses Pulver vom Schmp. 165°C (Zers.) erhalten wird.

### 12. 2-{[[4-(2-Furylmethylthio)-3-methyl-2-pyridinyl]methyl]thio}-1H-benz-imidazol-dihydrochlorid

Die Verbindung des Beispiels 1 läßt sich auch über das korrespondierende Dihydrochlorid herstellen indem man 2-Mercapto-1H-benzimidazol (1,5 g/10 mMol) in 30 ml Isopropanol mit 2-Chlormethyl-4-(2-furylmethylthio)-3-methylpyridin-hydrochlorid (10 mMol) 5 h unter Rückfluß erhitzt, abkühlt, vom ausgefallenen Feststoff filtriert und aus Isopropanol/Wasser umkristallisiert. Man erhält die Titelverbindung (90 % Ausbeute) als farblosen Feststoff vom Schmp. 208°C (Zers.).

### 13. 2-{[[4-(2-Furylmethylthio)-3-methyl-2-pyridinyl]methyl]thio}-5-trifluormethyl-1H-benzimidazol-dihydrochlorid

Nach der in Beispiel 12 angegebenen Arbeitsweise erhält man durch Umsetzung von 2-Chlormethyl-4-(2-furylmethylthio)-3-methylpyridin-hydrochlorid mit 2-Mercapto-5-trifluormethyl-1H-benzimidazol in Isopropanol die Titelverbindung als farblosen Feststoff; Schmp. 173°C (Zers.).

### 14. 2-{[[4-(2-Furylmethylthio)-3-methyl-2-pyridinyl]methyl]thio}-5-(2,2,2-trifluorethoxy)-1H-benzimidazol-dihydrochlorid

Nach der in Beispiel 12 angegebenen Arbeitsweise erhält man durch Umsetzung von 2-Chlormethyl-4-(2-furylmethylthio)-3-methylpyridin-hydrochlorid mit 2-Mercapto-5-(2,2,2-trifluorethoxy)-1H-benzimidazol in Isopropanol die Titelverbindung als farblosen Feststoff; Schmp. 161-163°C (Zers.).

### 15. 2,2-Difluor-6-[[(4-(2-furylmethylthio)-3-methyl-2-pyridinyl]methyl]-thio}-5H-[1,3]dioxolo-[4,5-f]benzimidazol-dihydrochlorid

Nach der in Beispiel 12 angegebenen Arbeitsweise erhält man durch Umsetzung von 2-Chlormethyl-4-(2-furylmethylthio)-3-methylpyridin-hydrochlorid mit 2,2-Difluor-6-mercapto-5H-[1,3]dioxolo-[4,5-f]benzimidazol in Isopropanol die Titelverbindung als farblosen Feststoff; Schmp. 180-183°C (Zers.).

### 16. 2-{[[4-(3,4-Dimethoxy-2-pyridinylmethylthio)-3-methyl-2-pyridinyl]-methyl]thio}-1H-benzimidazol

Nach der in Beispiel 1 angegebenen Arbeitsweise erhält man durch Umsetzung von 2-Mercapto-1H-benzimidazol mit 2-Chlormethyl-4-[(3,4-dimethoxy)-2-pyridinylmethylthio)]-3-methylpyridin-hydrochlorid in Ethanol unter Zusatz von Natronlauge ein gelbes Öl. Kristallisation aus Ethylacetat liefert die Titelverbindung als farblosen Feststoff vom Schmp. 165-167°C.

### 17. 2-{[[4-(2-Pyridinyl-2-ethylthio)-3-methyl-2-pyidinyl]methyl]thio}-1H-benzimidazol

Nach der in Beispiel 1 angegebenen Arbeitsweise erhält man durch Umsetzung von 2-Mercapto-lH-benzimidazol mit 2-Chlormethyl-4-(2-pyridinyl-2-ethylthio)-3-methylpyridin-hydrochlorid nach Kristallisation aus Diisopropylether die Titelverbindung; Schmp. 147-149°C.

### 18. 2-{[[4-(3-(Imidazol-1-yl)propylthio]-3-methyl-2-pyridinyl]methyl]-thio}-1H-benzimidazol

Nach der in Beispiel 1 angegebenen Arbeitsweise erhält man durch Umsetzung von 2-Mercapto-lH-benzimidazol mit 2-Chlormethyl-4-[3-(imidazol-1-yl)propylthio]-3-methylpyridin-hydrochlorid nach Kristallisation aus Diisopropylether die Titelverbindung als farblosen Feststoff; Schmp. 145-146°C.

### 19. 2-{[[4-[3-(2-Methylimidazol-1-yl)propylthio]-3-methyl-2-pyridinyl]-methyl]thio}-1H-benzimidazol

Nach der in Beispiel 1 beschriebenen Arbeitsweise erhält man die Titelverbindung aus 2-Chlormethyl-4-[3-(2-methylimidazol-1-yl)propylthio]-3-methyl-pyridin-hydrochlorid als gelbes Öl. Nach der in Beispiel 4 beschriebenen Arbeitsweise erhält man daraus ein Hydrochloridsalz der Titelverbindung vom Schmp. 212-215°C (Zers.).

### 20. 2-{[[4-(2-Furylmethylthio)-3-methyl-2-pyridinyl]methyl]sulfinyl}-1H-benzimidazol

2-{[[4-(2-Furylmethylthio)-3-methyl-2-pyridinyl]methyl]thio}-1H-benzimidazol (2 mMol) werden in 15 ml Dioxan + NaOH (6 mMol; 2 N) gelöst und mit 2,5 mMol 7-prozentiger Natriumhypochloritlösung bei 20°C versetzt. Man setzt Natriumthiosulfat zu, destilliert Dioxan ab, stellt den pH auf 9, extrahiert mit Dichlormethan und kristallisiert aus Dichlormethan/Diisopropylether. Man erhält die Titelverbindung (Ausbeute 80 %) vom Schmp. 173°C (Zers.) als farblosen Feststoff.

### Ausgangsverbindungen

### A. 2-Chlormethyl-4-(2-furylmethylthio)-3-methylpyridin-hydrochlorid

a) 2,3-Dimethyl-4-(2-furylmethylthio)pyridin-N-oxid
   Zu 50 ml trockenem Dioxan werden 6 g (60 %iges) NaH portionsweise zugegeben, es wird 15 Min. gerührt, 11,7 g (0,11 Mol) 2-Furylmethylmercaptan werden innerhalb von 20 Min. zudosiert und es wird erneut 30 Min. bis zur Beendigung der Gasentwicklung gerührt. Anschließend tropft man innerhalb von 20 Min. eine Lösung von 14,4 g (0,1 Mol) 4-Chlor-2,3-dimethylpyridin-N-oxid in 100 ml Dioxan zu, rührt die Reaktionsmischung 1 h bei RT, anschließend 1 h bei 70°C und danach noch 1 h bei 100°C. Nach beendeter Umsetzung läßt man abkühlen, verdünnt mit 500 ml Wasser und extrahiert 4 mal mit je 300 ml Ethylacetat. Die vereinigten organischen Phasen werden mit Wasser gewaschen, über Magnesiumsulfat getrocknet, eingeengt und durch Zugabe von Diisopropylether kristallisiert. Man erhält 18,8 g (80 % d.Th.) 2,3-Dimethyl-4-(2-furylmethylthio)-pyridin-N-oxid] vom Schmp. 111-112°C.
b) 2-Acetoxymethyl-4-(2-furylmethylthio)-3-methylpyridin
   18,0 g (0,77 Mol) des unter a) erhaltenden Produktes werden in 100 ml Essigsäureanhydrid erwärmt (100°C) und 2 h gerührt. Nach Einengen im Vakuum wird der braune, ölige Rückstand in einer Kugelrohrdestillationsapparatur destilliert. Man erhält 17,0 g 2-Acetoxymethyl-4-(2-furylmethylthio)-3-methylpyridin, das direkt weiter umgesetzt wird.
c) 4-(2-Furylmethylthio)-2-hydroxymethyl-3-methylpyridin
   Das Produkt aus b) (17,0 g) wird in 100 ml 2 n Natronlauge und 100 ml Isopropanol 2 h unter Rühren auf Rückflußtemperatur erhitzt, Isopropanol abdestilliert und der Rückstand 3 mal mit je 100 ml Dichlormethan extrahiert. Die vereinigten organischen Phasen werden mit Wasser gewaschen, über Kaliumcarbonat getrocknet, im Vakuum eingeengt und aus wenig Toluol kristallisiert. Man erhält 13,4 g (93 %) 4-(2-Furylmethylthio)-2-hydroxymethyl-3-methylpyridin als cremefarbenen Feststoff vom Schmp. 60-62°C.
d) 2-Chlormethyl-4-(2-furylmethylthio)-3-methylpyridin-hydrochlorid
   10,0 g (0,042 Mol) 4-(2-Furylmethylthio)-2-hydroxymethyl-3-methylpyridin werden in Dichlormethan (100 ml) gelöst, bei RT 1,2 Äquivalente Thionylchlorid zugetropft und 20 h bei RT gerührt. Man engt vollständig ein und erhält die Titelverbindung als öligen, allmählich kristallisierenden Rückstand, der gewünschtenfalls auch als Lösung in Ethanol direkt zur Umsetzung mit substituierten 2-Mercaptobenzimidazolen verwendet werden kann. Zur Reinigung wird aus heißem Isopropanol unter Zusatz von Aktivkohle umkristallisiert. Man erhält 9,0 g (74 % d.Th.) der Titelverbindung als farbloses Kristallisat vom Schmp. 159-161°C (Zers.).

### B) 2-Chlormethyl-3-methyl-4-[2-(4-methyl-5-thiazolyl)-ethylthio]pyridinhydrochlorid

a) 2,3-Dimethyl-4-[2-(4-methyl-5-thiazolyl)-ethylthio]-pyridin-N-oxid
   Nach der in Beispiel Aa) angegebenen Arbeitsweise erhält man durch Umsetzung von 4-Chlor-2,3-dimethylpyridin-N-oxid mit 5-(2-Mercaptoethyl)-4-methylthiazol in Gegenwart von Natriumhydrid 2,3-Dimethyl-4-[2-(4-methyl-5-thiazolyl)-ethylthio]-pyridin-N-oxid; Schmp.: 135-137°C (Ausbeute: 79 %).
b) 2-Acetoxymethyl-3-methyl-4-[2-(4-methyl-5-thiazolyl)-ethylthio]pyridin
   Nach der in Beispiel Ab) angegebenen Arbeitsweise erhält man aus Produkt Ba) 2-Acetoxymethyl-3-methyl-4-[2-(4-methyl-5-thiazolyl)-ethylthio]pyridin als gelbes Öl, das direkt weiter umgesetzt wird.
c) 2-Hydroxymethyl-3-methyl-4-[2-(4-methyl-5-thiazolyl)-ethylthio]pyridin
   Nach der unter Ac) angegebenen Arbeitsweise erhält man aus Produkt Bb) die Titelverbindung, die als Rohprodukt ohne Kristallisation direkt weiter umgesetzt wird.
d) 2-Chlormethyl-3-methyl-4-[2-(4-methyl-5-thiazolyl)-ethylthio]pyridinhydrochlorid
   Nach der unter Ad) angegebenen Arbeitsweise erhält man aus Produkt Bc) die Titelverbindung, die als Rohprodukt in Ethanol gelöst wird und direkt weiter umgesetzt wird.

### C) 2-Chlormethyl-4-{[(5-dimethylaminomethyl-2-furyl)methyl]thio}-3-methylpyridin-hydrochlorid

a) 4-([(5-Dimethylaminomethyl-2-furyl)methyl]thio)-2-hydroxymethyl-3-methylpyridin
   1,5 g (6,4 mmol) 5-(2-Furylmethylthio)-2-hydroxymethyl-3-methyl-pyridin (hergestellt gemäß Beispiel Ac) werden in 40 ml Acetonitril gelöst, mit 1,5 g (8,0 mmol) N,N-Dimethyl-methylenimmonium-iodid versetzt und 4 h bei 80°C gerührt. Nach Abdestillieren des Acetonitrils im Vakuum wird der Rückstand mit Wasser (10 ml) versetzt, mit Natriumcarbonatlösung auf pH10 gestellt und mit Ethylacetat extrahiert (3 x 20 ml). Die vereinigten organischen Phasen werden mit Wasser gewaschen, über Kaliumcarbonat getrocknet, eingeengt und der Rückstand wird an Kieselgel chromatographiert (Dichlormethan/Methanol/Triethylamin 4/1/0,1). Man erhält 1,06 g (57 %) der Titelverbindung als gelbes Öl.
   H NMR (CDCl₃): ppm 2.13 (s, 3H); 2.25 (s, 6H); 3.42 (s, 2H); 4.19 (s, 2H); 4.68 (s, 2H); 6.10-6.19 (AB-System, 2H); 7.15 (d, J=5.4 Hz, 1H); 8,23 (d, 1H).
   Nach Auflösung in Diethylether erhält man durch Zugabe von etherischer Salzsäure die Titelverbindung als farbloses, hygroskopisches Dihydrochlorid. Zers. ab 90°C.
b) 2-Chlormethyl-4-{[(5-dimethylaminomethyl-2-furyl)methyl]thio}-3-methylpyridin-dihydrochlorid
   Nach der unter Ad) angegebenen Arbeitsweise erhält man ausgehend von der Verbindung des Beispiels Ca) die Titelverbindung als Rohprodukt, das in Ethanol gelöst und direkt weiter umgesetzt wird. Kristallisation aus Isopropanol liefert ein kristallines, farbloses Dihydrochlorid; Schmp. ab 185°C (Zers.).

Auf analoge Weise - wie beispielsweise in den Beispielen Aa) bis Ad) beschrieben - erhält man die Hydrochloride der folgenden Verbindungen:

2-Chlormethyl-3-methyl-4-(2-thienylmethylthio)-pyridin, 2-Chlormethyl-3-methyl-4-(3-thienylmethylthio)-pyridin, 2-Chlormethyl-3-methoxy-4-(2-thienylmethylthio)-pyridin, 2-Chlormethyl-4-(2-thienylmethylthio)-pyridin, 2-Chlormethyl-4-(2-furylmethylthio)-pyridin, 2-Chlormethyl-4-[(3,4-dimethoxy)-2-pyridinyl-methylthio)]-3-methylpyridin und 2-Chlormethyl-4-[2-pyridinyl-2-ethylthio]-3-methylpyridin.

### D) 2-Chlormethyl-4-(2-furylmethylthio)-3-methoxypyridin-hydrochlorid

Nach der in bei Aa) bis Ac) beschriebenen Arbeitsweise erhält man ausgehend von 4-Chlor-3-methoxy-2-methylpyridin-N-oxid das Zwischenprodukt 4-(2-Furylmethylthio)-2-hydroxymethyl-3-methoxypyridin; Schmp. 56-58°C. Chlorierung mit Thionylchlorid nach der in Beispiel Ad) beschriebenen Arbeitsweise liefert die Titelverbindung als beiges Pulver; Schmp. 135°C (Zers.).

### Gewerbliche Anwendbarkeit

Die ausgezeichnete Wirksamkeit von Verbindungen der Formel I und ihren Salzen gegen Helicobacter-Bakterien gestattet ihren Einsatz in der Humanmedizin als Wirkstoffe für die Behandlung von Krankheiten, die auf Helicobacter-Bakterien beruhen.

Ein weiterer Gegenstand der Erfindung ist daher ein Verfahren zur Behandlung von Säugern, insbesondere Menschen, die an Krankheiten erkrankt sind, die auf Helicobacter-Bakterien beruhen. Das Verfahren ist dadurch gekennzeichnet, daß man dem erkrankten Individuum eine therapeutisch wirksame und pharmakologisch verträgliche Menge einer oder mehrerer Verbindungen der Formel I und/oder ihrer pharmakologisch verträglichen Salze verabreicht.

Gegenstand der Erfindung sind außerdem die Verbindungen der Formel I und ihre pharmakologisch verträglichen Salze zur Anwendung bei der Behandlung von Krankheiten, die auf Helicobacter-Bakterien beruhen.

Ebenso umfaßt die Erfindung die Verwendung von Verbindungen der Formel I und ihren pharmakologisch verträglichen Salzen bei der Herstellung von Arzneimitteln, die zur Bekämpfung solcher Krankheiten eingesetzt werden, die auf Helicobacter-Bakterien beruhen.

Ein weiterer Gegenstand der Erfindung sind Arzneimittel zur Bekämpfung von Helicobacter-Bakterien, die eine oder mehrere Verbindungen der allgemeinen Formel I und/oder ihre pharmakologisch verträglichen Salze enthalten.

Von den Helicobacter-Stämmen, gegenüber denen sich die Verbindungen der Formel I als wirksam erweisen, sei insbesondere der Stamm Helicobacter pylori erwähnt.

Die Arzneimittel werden nach an sich bekannten, dem Fachmann geläufigen Verfahren hergestellt: Als Arzneimittel werden die pharmakologisch wirksamen Verbindungen der Formel I und ihre Salze (=Wirkstoffe) entweder als solche, oder vorzugsweise in Kombination mit geeigneten pharmazeutischen Hilfsstoffen z.B. in Form von Tabletten, Dragees, Kapseln, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder Permeationspromotoren und Komplexbildner (z.B. Cyclodextrine) verwendet werden.

Die Wirkstoffe können beispielsweise parenteral (z.B. intravenös) oder insbesondere oral appliziert werden.

Im allgemeinen werden in der Humanmedizin die Wirkstoffe in einer Tagesdosis von etwa 0,2 bis 50, vorzugsweise 1 bis 30 mg/kg Körpergewicht, gegebenenfalls in Form mehrerer, vorzugsweise 2 bis 6 Einzelgaben zur Erzielung des gewünschten Ergebnisses verabreicht.

In diesem Zusammenhang ist als erfindungswesentlicher Aspekt besonders zu erwähnen, daß sich die Verbindungen der Formel I, in denen n die Zahl 0 bedeutet, gegenüber Helicobacter-Bakterien bereits bei Verabfolgung solcher Dosen als wirksam erweisen, die unterhalb der Dosen liegen, die zur Erzielung einer - therapeutischen Zwecken genügenden - Magensäuresekretionshemmung eingesetzt werden müßten.

### Biologische Untersuchungen

Die Verbindungen der Formel I wurden bezüglich ihrer Wirksamkeit gegenüber Helicobacter pylori in Anlehnung an die von Tomoyuki Iwahi et al. (Antimicrobial Agents and Chemotherapy, 1991, 490-496) beschriebene Methodik unter Verwendung von Columbia-agar (Oxoid) und bei einer Wachstumsperiode von 4 Tagen untersucht. Für die untersuchten Verbindungen ergaben sich hierbei die in der nachfolgenden Tabelle 2 aufgeführten MIC-Werte (die angegebenen Nummern der Verbindungen stimmen mit den Verbindungsnummern in der Beschreibung überein).

**TABELLE 2**

| Verbindung Nr. | MIC-Wert (µg/ml) |
|---|---|
| 1 | ≤ 1,0 |
| 2 | ≤ 1,0 |
| 5 | ≤ 1,0 |
| 6 | ≤ 1,0 |
| 8 | ≤ 1,0 |
| 12 | ≤ 1,0 |
| 16 | ≤ 1,0 |
| 17 | ≤ 1,0 |
| 18 | ≤ 1,0 |
| 19 | ≤ 1,0 |
| 20 | ≤ 1,0 |

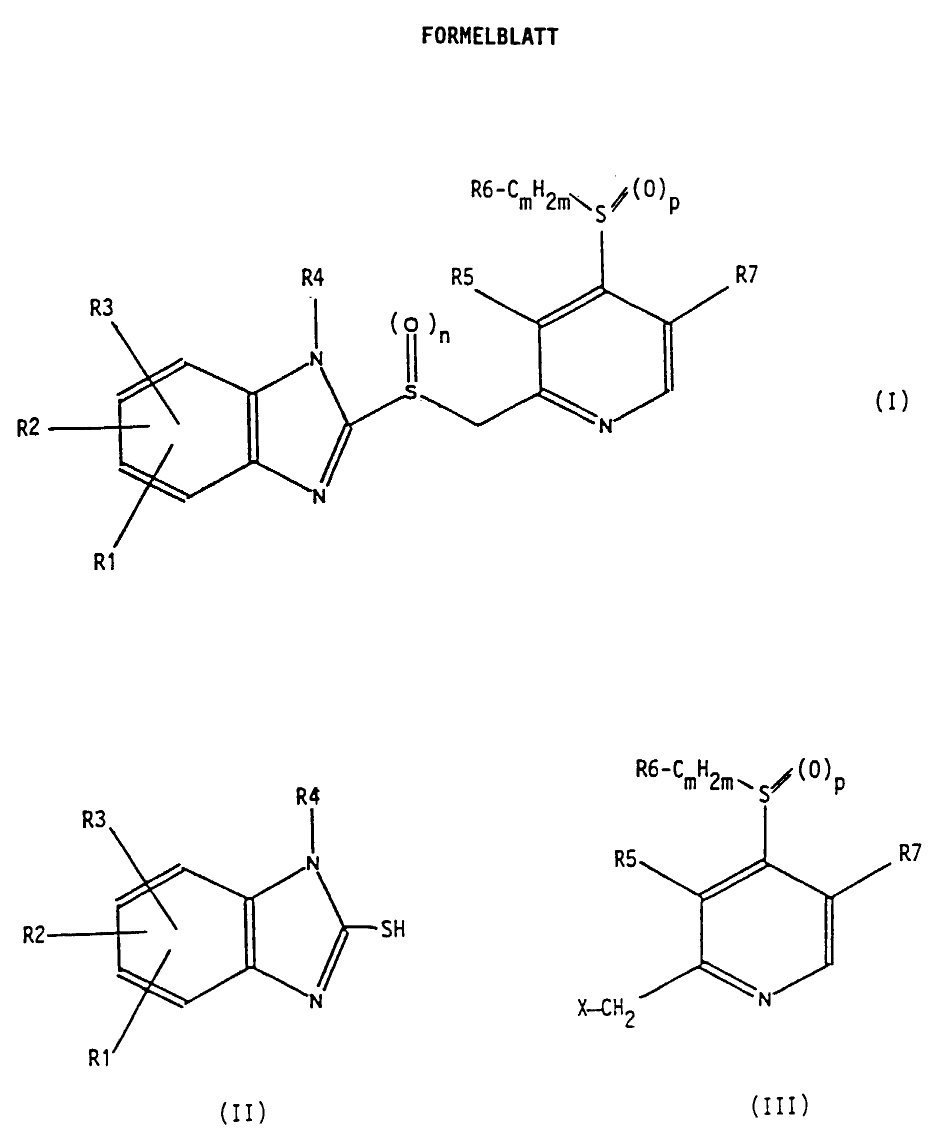

## Patentansprüche

1. Verbindungen der Formel I, 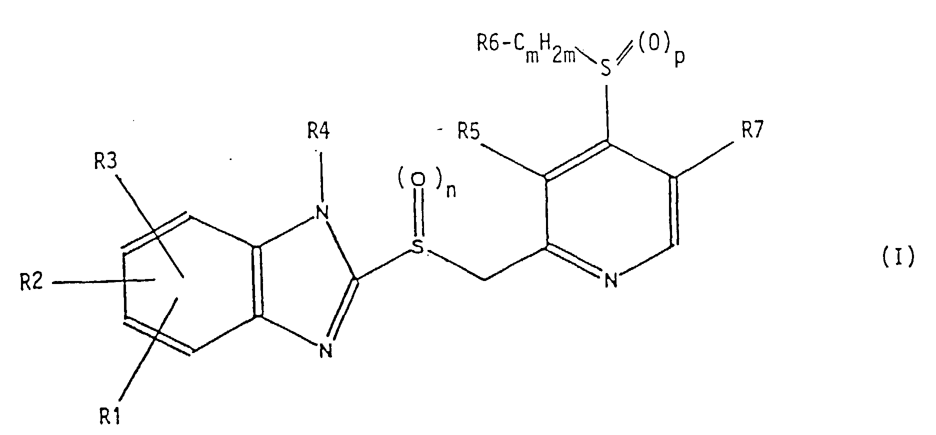 worin
R1 Wasserstoff, 1-4C-Alkyl oder 1-4C-Alkoxy bedeutet,
R2 Wasserstoff, 1-4C-Alkyl, 1-4C-Alkoxy, Halogen, Trifluormethyl, ganz oder überwiegend durch Fluor substituiertes 1-4C-Alkoxy, Chlordifluormethoxy, 2-Chlor-1,1,2-trifluorethoxy oder gemeinsam mit R3 gewünschtenfalls ganz oder teilweise durch Fluor substituiertes 1-2C-Alkylendioxy oder Chlortrifluorethylendioxy bedeutet,
R3 Wasserstoff, ganz oder überwiegend durch Fluor substituiertes 1-4C-Alkoxy, Chlordifluormethoxy, 2-Chlor-1,1,2-trifluorethoxy oder gemeinsam mit R2 gewünschtenfalls ganz oder teilweise durch Fluor substituiertes 1-2C-Alkylendioxy oder Chlortrifluorethylendioxy bedeutet,
R4 Wasserstoff oder 1-4C-Alkyl bedeutet,
R5 Wasserstoff, 1-4C-Alkyl oder 1-4C-Alkoxy bedeutet,
R6 einen durch R8 und R9 substituierten Heterocyclus bedeutet, der ausgewählt ist aus der Gruppe bestehend aus Furan, Thiophen, Pyrrol, Oxazol, Isoxazol, Thiazol, Isothiazol, Imidazol, Pyrazol, 1,2,3-Triazol, 1,2,4-Triazol, Tetrazol, 1,3,4-Thiadiazol, 1,2,4-Thiadiazol, Pyrimidin und Pyridin,
R7 Wasserstoff, 1-4C-Alkyl oder 1-4C-Alkoxy bedeutet,
R8 Wasserstoff, 1-4C-Alkyl, 1-4C-Alkoxy, Halogen, durch R10 substituiertes 1-4C-Alkyl oder -N(R11)R12 bedeutet,
R9 Wasserstoff, 1-4C-Alkyl, 1-4C-Alkoxy oder Halogen bedeutet,
R10 Hydroxy, 1-4C-Alkoxy oder -N(R11)R12 bedeutet, wobei
R11 Wasserstoff, 1-4C-Alkyl oder -CO-R13 und
R12 Wasserstoff oder 1-4C-Alkyl bedeutet,
R13 Wasserstoff, 1-4C-Alkyl oder 1-4C-Alkoxy bedeutet,
m eine Zahl von 1 bis 7 bedeutet,
n die Zahl 0 oder 1 bedeutet und
p die Zahl 0 oder 1 bedeutet,
und ihre Salze.

2. Verbindungen der Formel I nach Anspruch 1, worin R6 einen durch R8 und R9 substituierten Heterocyclus bedeutet, der ausgewählt ist aus der Gruppe bestehend aus Furan, Thiophen, Pyrrol, Oxazol, Isoxazol, Thiazol, Isothiazol, Imidazol, Pyrazol, 1,2,3-Triazol, 1,2,4-Triazol, Tetrazol, 1,3,4-Thiadiazol und 1,2,4-Thiadiazol, und ihre Salze.

3. Verbindungen der Formel I nach Anspruch 1, worin
R1 Wasserstoff bedeutet,
R2 Wasserstoff, 1-4C-Alkoxy, Trifluormethyl, ganz oder überwiegend durch Fluor substituiertes 1-4C-Alkoxy, oder gemeinsam mit R3 ganz oder teilweise durch Fluor substituiertes 1-2C-Alkylendioxy bedeutet,
R3 Wasserstoff oder gemeinsam mit R2 ganz oder teilweise durch Fluor substituiertes 1-2C-Alkylendioxy bedeutet,
R4 Wasserstoff bedeutet,
R5 Wasserstoff, 1-4C-Alkyl oder 1-4C-Alkoxy bedeutet,
R6 einen durch R8 und R9 substituierten Heterocyclus bedeutet, der ausgewählt ist aus der Gruppe bestehend aus Furan, Thiophen, Thiazol, Imidazol und Pyridin,
R7 Wasserstoff bedeutet,
R8 Wasserstoff, 1-4C-Alkyl, 1-4C-Alkoxy oder durch R10 substituiertes 1-4C-Alkyl bedeutet,
R9 Wasserstoff oder 1-4C-Alkoxy bedeutet,
R10 -N(R11)R12 bedeutet, wobei
R11 1-4C-Alkyl und
R12 1-4C-Alkyl bedeutet,
m eine Zahl von 1 bis 3 bedeutet,
n die Zahl 0 oder 1 bedeutet und
p die Zahl 0 bedeutet,
und ihre Salze.

4. Verbindungen der Formel I nach Anspruch 1, worin
R1 Wasserstoff bedeutet,
R2 Wasserstoff oder Methoxy bedeutet,
R3 Wasserstoff bedeutet,
R4 Wasserstoff bedeutet,
R5 Wasserstoff, 1-4C-Alkyl oder 1-4C-Alkoxy bedeutet,
R6 einen durch R8 und R9 substituierten Heterocyclus bedeutet, der ausgewählt ist aus der Gruppe bestehend aus Furan, Thiophen, Thiazol, Imidazol und Pyridin,
R7 Wasserstoff bedeutet,
R8 Wasserstoff, 1-4C-Alkyl, 1-4C-Alkoxy oder durch R10 substituiertes 1-4C-Alkyl bedeutet,
R9 Wasserstoff oder 1-4C-Alkoxy bedeutet,
R10 -N(R11)R12 bedeutet, wobei
R11 1-4C-Alkyl und
R12 1-4C-Alkyl bedeutet,
m eine Zahl von 1 bis 3 bedeutet,
n die Zahl 0 bedeutet und
p die Zahl 0 bedeutet,
und ihre Salze.

5. Verbindungen der Formel I nach Anspruch 1, worin
R1 Wasserstoff bedeutet,
R2 Wasserstoff, Halogen, Methoxy, Difluormethoxy oder Trifluormethyl bedeutet,
R3 Wasserstoff bedeutet,
R4 Wasserstoff bedeutet,
R5 Wasserstoff, 1-4C-Alkyl oder 1-4C-Alkoxy bedeutet,
R6 einen durch R8 und R9 substituierten Heterocyclus bedeutet, der ausgewählt ist aus der Gruppe bestehend aus Furan, Thiophen, Thiazol, Imidazol, Pyrrol, Oxazol und Pyrazol,
R7 Wasserstoff bedeutet,
R8 Wasserstoff, 1-4C-Alkyl, Guanidino, durch R10 substituiertes 1-4C-Alkyl oder -N(R11)R12 bedeutet,
R9 Wasserstoff oder 1-4C-Alkyl bedeutet,
R10 -N(R11)R12 bedeutet,
R11 Wasserstoff, 1-4C-Alkyl oder -CO-R13 bedeutet,
R12 Wasserstoff oder 1-4C-Alkyl bedeutet,
R13 Wasserstoff, 1-4C-Alkyl oder 1-4C-Alkoxy bedeutet,
m eine Zahl von 1 bis 3 bedeutet,
n die Zahl 0 bedeutet und
p die Zahl 0 bedeutet
und ihre Salze.

6. Verbindungen der Formel I nach Anspruch 1, worin
R1 Wasserstoff bedeutet,
R2 Wasserstoff oder Methoxy bedeutet,
R3 Wasserstoff bedeutet,
R4 Wasserstoff bedeutet,
R5 Wasserstoff, 1-4C-Alkyl oder 1-4C-Alkoxy bedeutet,
R6 einen durch R8 und R9 substituierten Heterocyclus bedeutet, der ausgewählt ist aus der Gruppe bestehend aus Furan, Thiophen, Thiazol und Imidazol,
R7 Wasserstoff bedeutet,
R8 Wasserstoff, Guanidino, durch R10 substituiertes Methyl oder -N(R11)R12 bedeutet,
R9 Wasserstoff oder 1-4C-Alkyl bedeutet,
R10 -N(R11)R12 bedeutet,
R11 Wasserstoff, 1-4C-Alkyl oder -CO-R13 bedeutet,
R12 Wasserstoff oder 1-4C-Alkyl bedeutet,
R13 Wasserstoff, 1-4C-Alkyl oder 1-4C-Alkoxy bedeutet,
m die Zahl 1 oder 2 bedeutet,
n die Zahl 0 bedeutet und
p die Zahl 0 bedeutet
und ihre Salze.

7. Verfahren zur Herstellung der Verbindungen der Formel I nach Anspruch 1, worin R1, R2, R3, R4, R5, R6, R7, m, n und p die in Anspruch 1 angegebenen Bedeutungen haben, und ihrer Salze, dadurch gekennzeichnet, daß man Mercaptobenzimidazole der Formel II 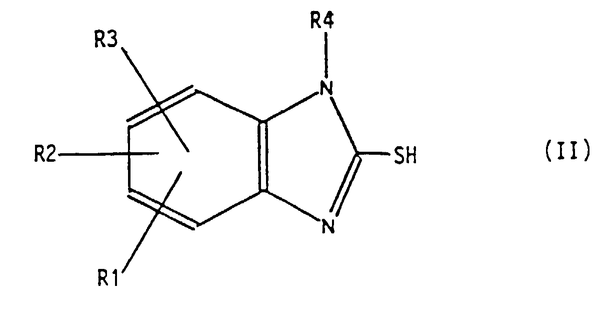 worin R1, R2, R3 und R4 die in Anspruch 1 angegebenen Bedeutungen haben, mit Picolinderivaten III 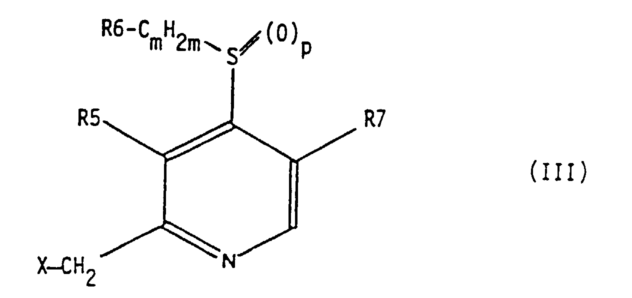 worin R5, R6, R7, m und p die in Anspruch 1 angegebenen Bedeutungen haben und X eine geeignete Abgangsgruppe darstellt, umsetzt und (falls Verbindungen der Formel I mit n=1 die gewünschten Endprodukte sind) anschliessend die erhaltenen 2-Benzimidazolyl-2-pyridylmethyl-sulfide der Formel I mit n=0 oxydiert und/oder gewünschtenfalls in die Salze überführt.

8. Arzneimittel enthaltend eine oder mehrere Verbindungen der Formel I nach Anspruch 1 und/oder ihre pharmakologisch verträglichen Salze.

9. Verbindungen der Formel I nach Anspruch 1 und/oder ihre pharmakologisch verträglichen Salzen zur Anwendung bei der Bekämpfung von Helicobacter-Bakterien.

10. Verwendung von Verbindungen der Formel I nach Anspruch 1 und ihren pharmakologisch verträglichen Salzen zur Herstellung von Arzneimitteln für die Bekämpfung von Helicobacter-Bakterien.

## Claims

1. Compounds of the formula I 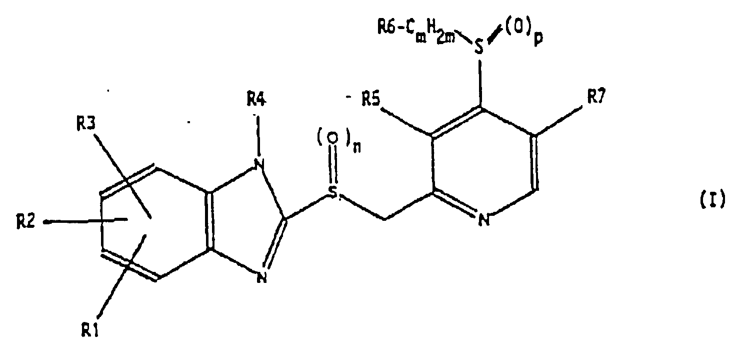 in which
R1 is hydrogen, 1-4C-alkyl or 1-4C-alkoxy,
R2 is hydrogen, 1-4C-alkyl, 1-4C-alkoxy, halogen, trifluoromethyl, fully or predominantly fluorine-substituted 1-4C-alkoxy, chlorodifluoromethoxy, 2-chloro-1,1,2-trifluoroethoxy, or together with R3 1-2C-alkylenedioxy which, if desired, is fully or partially substituted by fluorine, or chlorotrifluoroethylenedioxy,
R3 is hydrogen, 1-4C-alkoxy which is fully or predominantly substituted by fluorine, or is chlorodifluoromethoxy, 2-chloro-1,1,2-trifluoroethoxy, or together with R2 1-2C-alkylenedioxy which, if desired, is fully or partially substituted by fluorine, or chlorotrifluoroethylenedioxy,
R4 is hydrogen or 1-4C-alkyl,
R5 is hydrogen, 1-4C-alkyl or 1-4C-alkoxy,
R6 is a heterocycle selected from the group consisting of furan, thiophene, pyrrole, oxazole, isoxazole, thiazole, isothiazole, imidazole, pyrazole, 1,2,3-triazole, 1,2,4-triazole, tetrazole, 1,3,4-thiadiazole, 1,2,4-thiadiazole, pyrimidine and pyridine, each of which is substituted by R8 and R9,
R7 is hydrogen, 1-4C-alkyl or 1-4C-alkoxy,
R8 is hydrogen, 1-4C-alkyl, 1-4C-alkoxy, halogen, 1-4C-alkyl which is substituted by R10, or -N(R11)R12,
R9 is hydrogen, 1-4C-alkyl, 1-4C-alkoxy or halogen,
R10 is hydroxyl, 1-4C-alkoxy or -N(R11)R12, where
R11 is hydrogen, 1-4C-alkyl or -CO-R13 and
R12 is hydrogen or 1-4C-alkyl,
R13 is hydrogen, 1-4C-alkyl or 1-4C-alkoxy,
m is a number from 1 to 7,
n is the number 0 or 1 and
p is the number 0 or 1,
and their salts.

2. Compounds of the formula I as claimed in claim 1, in which R6 is a heterocycle selected from the group consisting of furan, thiophene, pyrrole, oxazole, isoxazole, thiazole, isothiazole, imidazole, pyrazole, 1,2,3-triazole, 1,2,4-triazole, tetrazole, 1,3,4-thiadiazole and 1,2,4-thiadiazole, and which is substituted by R8 and R9, and their salts.

3. Compounds of the formula I as claimed in claim 1, in which
R1 is hydrogen,
R2 is hydrogen, 1-4C-alkoxy, trifluoromethyl, 1-4C-alkoxy which is fully or predominantly substituted by fluorine, or together with R3 is 1-2C-alkylenedioxy which is fully or partially substituted by fluorine,
R3 is hydrogen or together with R2 is 1-2C-alkylenedioxy which is fully or partially substituted by fluorine,
R4 is hydrogen,
R5 is hydrogen, 1-4C-alkyl or 1-4C-alkoxy,
R6 is a heterocycle which is selected from the group consisting of furan, thiophene, thiazole, imidazole and pyridine and which is substituted by R8 and R9,
R7 is hydrogen,
R8 is hydrogen, 1-4C-alkyl or 1-4C-alkoxy or 1-4C-alkyl which is substituted by R10,
R9 is hydrogen or 1-4C-alkoxy,
R10 is -N(R11)R12, where
R11 is 1-4C-alkyl and
R12 is 1-4C-alkyl,
m is a number from 1 to 3,
n is the number 0 or 1 and
p is the number 0,
and their salts.

4. Compounds of the formula I as claimed in claim 1, in which
R1 is hydrogen,
R2 is hydrogen or methoxy,
R3 is hydrogen,
R4 is hydrogen,
R5 is hydrogen, 1-4C-alkyl or 1-4C-alkoxy,
R6 is a heterocycle which is selected from the group consisting of furan, thiophene, thiazole, imidazole and pyridine and which is substituted by R8 and R9,
R7 is hydrogen,
R8 is hydrogen, 1-4C-alkyl or 1-4C-alkoxy or 1-4C-alkyl which is substituted by R10,
R9 is hydrogen or 1-4C-alkoxy,
R10 is -N(R11)R12, where
R11 is 1-4C-alkyl and
R12 is 1-4C-alkyl,
m is a number from 1 to 3,
n is the number 0 and
p is the number 0,
and their salts.

5. Compounds of the formula I as claimed in claim 1, in which
R1 is hydrogen,
R2 is hydrogen, halogen, methoxy, difluoromethoxy or trifluoromethyl,
R3 is hydrogen,
R4 is hydrogen,
R5 is hydrogen, 1-4C-alkyl or 1-4C-alkoxy,
R6 is a heterocycle which is selected from the group consisting of furan, thiophene, thiazole, imidazole, pyrrole, oxazole and pyrazole and which is substituted by R8 and R9,
R7 is hydrogen,
R8 is hydrogen, 1-4C-alkyl, guanidino, 1-4C-alkyl which is substituted by R10, or -N(R11)R12,
R9 is hydrogen or 1-4C-alkyl,
R10 is -N(R11)R12,
R11 is hydrogen, 1-4C-alkyl or -CO-R13,
R12 is hydrogen or 1-4C-alkyl,
R13 is hydrogen, 1-4C-alkyl or 1-4C-alkoxy,
m is a number from 1 to 3,
n is the number 0 and
p is the number 0,
and their salts.

6. Compounds of the formula I as claimed in claim 1, in which
R1 is hydrogen,
R2 is hydrogen or methoxy,
R3 is hydrogen,
R4 is hydrogen,
R5 is hydrogen, 1-4C-alkyl or 1-4C-alkoxy,
R6 is a heterocycle which is selected from the group consisting of furan, thiophene, thiazole and imidazole and which is substituted by R8 and R9,
R7 is hydrogen,
R8 is hydrogen, guanidino, methyl which is substituted by R10, or -N(R11)R12,
R9 is hydrogen or 1-4C-alkyl,
R10 is -N(R11)R12,
R11 is hydrogen, 1-4C-alkyl or -CO-R13,
R12 is hydrogen or 1-4C-alkyl,
R13 is hydrogen, 1-4C-alkyl or 1-4C-alkoxy,
m is the number 1 or 2,
n is the number 0 and
p is the number 0,
and their salts.

7. A process for the preparation of the compounds of the formula I as claimed in claim 1, in which R1, R2, R3, R4, R5, R6, R7, m, n and p are as defined in claim 1, and of the salts thereof, which comprises reacting mercaptobenzimidazoles of the formula II 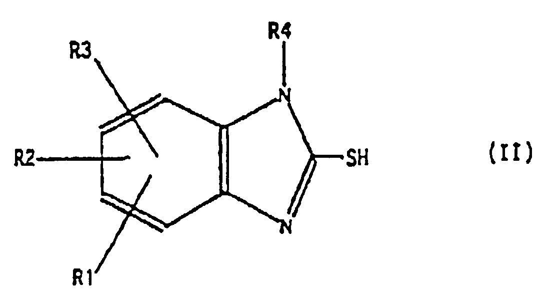 in which R1, R2, R3 and R4 are as defined in claim 1, with picoline derivatives III 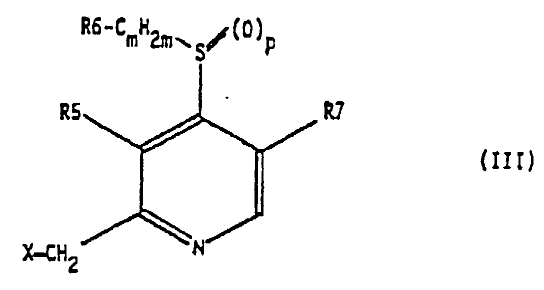 in which R5, R6, R7, m and p are as defined in claim 1 and X is a suitable leaving group, and (if the desired end products are compounds of the formula I where n=1) subsequently oxidizing the resulting 2-benzimidazolyl-2-pyridylmethyl sulfides of the formula I where n=0 and/or, if desired, converting them into the salts.

8. A drug product comprising one or more compounds of the formula I as claimed in claim 1 and/or the pharmacologically acceptable salts thereof.

9. Compounds of the formula I as claimed in Claim 1 and/or the pharmacologically acceptable salts thereof for the application in the control of HelicoBacter bacteria.

10. The use of compounds of the formula I as claimed in claim 1 and the pharmacologically acceptable salts thereof for the preparation of drug products for the control of Helicobacter bacteria.

## Revendications

1. Composés de formule I : 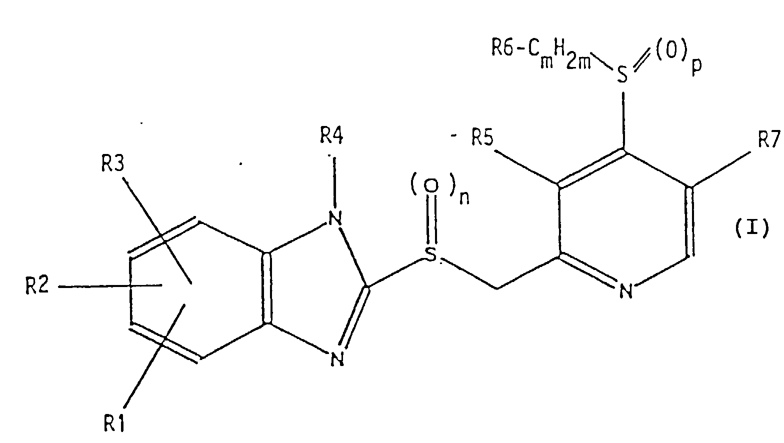 dans laquelle
R1 représente un atome d'hydrogène, un groupe alkyle à 1-4C ou un groupe alcoxy à 1-4C,
R2 représente un atome d'hydrogène ou d'halogène, un groupe alkyle à 1-4C, un groupe alcoxy à 1-4C, un groupe trifluorométhyle, un groupe alcoxy à 1-4C totalement ou principalement substitué par du fluor, un groupe chlorodifluorométhoxy ou un groupe 2-chlore-1,1,2-trifluoroéthoxy, ou bien R2 forme avec R3 un groupe alkylènedioxy à 1-2C, éventuellement substitué partiellement ou totalement par du fluor, ou un groupe chlorotrifluoroéthylènedioxy,
R3 représente un atome d'hydrogène, un groupe alcoxy à 1-4C totalement ou principalement substitué par du fluor, un groupe chlorodifluorométhoxy ou un groupe 2-chlore-1,1,2-trifluoroéthoxy, ou bien R3 forme avec R2 un groupe alkylènedioxy à 1-2C, éventuellement substitué partiellement ou totalement par du fluor, ou un groupe chlorotrifluoroéthylènedioxy,
R4 représente un atome d'hydrogène ou un groupe alkyle à 1-4C,
R5 représente un atome d'hydrogène, un groupe alkyle à 1-4C ou un groupe alcoxy à 1-4C,
R6 représente un hétérocycle substitué par R8 et R9, qui est choisi dans le groupe constitué du furanne, du thiophène, du pyrrole, de l'oxazole, de l'isoxazole, du thiazole, de l'isothiazole, de l'imidazole, du pyrazole, du 1,2,3-triazole, du 1,2,4-triazole, du tétrazole, du 1,3,4-thiadiazole, du 1,2,4-thiadiazole, de la pyrimidine et de la pyridine,
R7 représente un atome d'hydrogène, un groupe alkyle à 1-4C ou un groupe alcoxy à 1-4C,
R8 représente un atome d'hydrogène ou d'halogène, un groupe alkyle à 1-4C, un groupe alcoxy à 1-4C, un groupe alkyle à 1-4C substitué par R10 ou un groupe -N(R11)R12,
R9 représente un atome d'hydrogène ou d'halogène, un groupe alkyle à 1-4C ou un groupe alcoxy à 1-4C,
R10 représente un groupe hydroxy, un groupe alcoxy à 1-4C ou un groupe -N(R11)R12,
R11 représente un atome d'hydrogène, un groupe alkyle à 1-4C ou un groupe -CO-R13,
R12 représente un atome d'hydrogène ou un groupe alkyle à 1-4C,
R13 représente un atome d'hydrogène, un groupe alkyle à 1-4C ou un groupe alcoxy à 1-4C,
m représente un nombre de 1 à 7,
n représente le nombre 0 ou 1 et
p représente le nombre 0 ou 1,
et leurs sels.

2. Composés de formule I selon la revendication 1, dans lesquels R6 représente un hétérocycle substitué par R8 et R9, qui est choisi dans le groupe constitué du furanne, du thiophène, du pyrrole, de l'oxazole, de l'isoxazole, du thiazole, de l'isothiazole, de l'imidazole, du pyrazole, du 1,2,3-triazole, du 1,2,4-triazole, du tétrazole, du 1,3,4-thiadiazole et du 1,2,4-thiadiazole, et leurs sels.

3. Composés de formule I selon la revendication 1, dans lesquels
R1 représente un atome d'hydrogène,
R2 représente un atome d'hydrogène, un groupe alcoxy à 1-4C, un groupe trifluorométhyle ou un groupe alcoxy à 1-4C substitué totalement ou principalement par du fluor, ou bien R2 forme avec R3 un groupe alkylènedioxy à 1-2C, substitué totalement ou partiellement par du fluor,
R3 représente un atome d'hydrogène ou forme avec R2 un groupe alkylènedioxy à 1-2C, substitué totalement ou partiellement par du fluor,
R4 représente un atome d'hydrogène,
R5 représente un atome d'hydrogène, un groupe alkyle à 1-4C ou un groupe alcoxy à 1-4C,
R6 représente un hétérocycle substitué par R8 et R9, qui est choisi dans le groupe constitué du furanne, du thiophène, du thiazole, de l'imidazole et de la pyridine,
R7 représente un atome d'hydrogène,
R8 représente un atome d'hydrogène, un groupe alkyle à 1-4C, un groupe alcoxy à 1-4C ou un groupe alkyle à 1-4C substitué par R10,
R9 représente un atome d'hydrogène ou un groupe alcoxy à 1-4C,
R10 représente un groupe -N(R11)R12,
R11 représente un groupe alkyle à 1-4C,
R12 représente un groupe alkyle à 1-4C,
m représente un nombre de 1 à 3,
n représente le nombre 0 ou 1 et
p représente le nombre 0,
et leurs sels.

4. Composés de formule I selon la revendication 1, dans lesquels
R1 représente un atome d'hydrogène,
R2 représente un atome d'hydrogène ou un groupe méthoxy,
R3 représente un atome d'hydrogène,
R4 représente un atome d'hydrogène,
R5 représente un atome d'hydrogène, un groupe alkyle à 1-4C ou un groupe alcoxy à 1-4C,
R6 représente un hétérocycle substitué par R8 et R9, qui est choisi dans le groupe constitué du furanne, du thiophène, du thiazole, de l'imidazole et de la pyridine,
R7 représente un atome d'hydrogène,
R8 représente un atome d'hydrogène, un groupe alkyle à 1-4C, un groupe alcoxy à 1-4C ou un groupe alkyle à 1-4C substitué par R10,
R9 représente un atome d'hydrogène ou un groupe alcoxy à 1-4C,
R10 représente un groupe -N(R11)R12,
R11 représente un groupe alkyle à 1-4C,
R12 représente un groupe alkyle à 1-4C,
m représente un nombre de 1 à 3,
n représente le nombre 0 et
p représente le nombre 0,
et leurs sels.

5. Composés de formule I selon la revendication 1, dans lesquels
R1 représente un atome d'hydrogène,
R2 représente un atome d'hydrogène ou d'halogène, ou un groupe méthoxy, difluorométhoxy ou trifluorométhyle,
R3 représente un atome d'hydrogène,
R4 représente un atome d'hydrogène,
R5 représente un atome d'hydrogène, un groupe alkyle à 1-4C ou un groupe alcoxy à 1-4C,
R6 représente un hétérocycle substitué par R8 et R9, qui est choisi dans le groupe constitué du furanne, du thiophène, du thiazole, de l'imidazole, du pyrrole, de l'oxazole et du pyrazole,
R7 représente un atome d'hydrogène,
R8 représente un atome d'hydrogène, un groupe alkyle à 1-4C, un groupe guanidino, un groupe alkyle à 1-4C substitué par R10 ou un groupe -N(R11)R12,
R9 représente un atome d'hydrogène ou un groupe alkyle à 1-4C,
R10 représente un groupe -N(R11)R12,
R11 représente un atome d'hydrogène, un groupe alkyle à 1-4C ou un groupe -CO-R13,
R12 représente un atome d'hydrogène ou un groupe alkyle à 1-4C,
R13 représente un atome d'hydrogène, un groupe alkyle à 1-4C ou un groupe alcoxy à 1-4C,
m représente un nombre de 1 à 3,
n représente le nombre 0 et
p représente le nombre 0,
et leurs sels.

6. Composés de formule I selon la revendication 1, dans lesquels
R1 représente un atome d'hydrogène,
R2 représente un atome d'hydrogène ou un groupe méthoxy,
R3 représente un atome d'hydrogène,
R4 représente un atome d'hydrogène,
R5 représente un atome d'hydrogène, un groupe alkyle à 1-4C ou un groupe alcoxy à 1-4C,
R6 représente un hétérocycle substitué par R8 et R9, qui est choisi dans le groupe constitué du furanne, du thiophène, du thiazole et de l'imidazole,
R7 représente un atome d'hydrogène,
R8 représente un atome d'hydrogène, un groupe guanidino, un groupe méthyle substitué par R10 ou un groupe -N(R11)R12,
R9 représente un atome d'hydrogène ou un groupe alkyle à 1-4C,
R10 représente un groupe -N(R11)R12,
R11 représente un atome d'hydrogène, un groupe alkyle à 1-4C ou un groupe -CO-R13,
R12 représente un atome d'hydrogène ou un groupe alkyle à 1-4C,
R13 représente un atome d'hydrogène, un groupe alkyle à 1-4C ou un groupe alcoxy à 1-4C,
m représente le nombre de 1 ou 2,
n représente le nombre 0 et
p représente le nombre 0,
et leurs sels.

7. Procédé de préparation des composés de formule I selon la revendication 1, dans laquelle R1, R2, R3, R4, R5, R6, R7, m, n et p ont les significations indiquées dans la revendication 1, et leurs sels, caractérisé en ce que l'on fait réagir les mercaptobenzimidazoles de formule II : 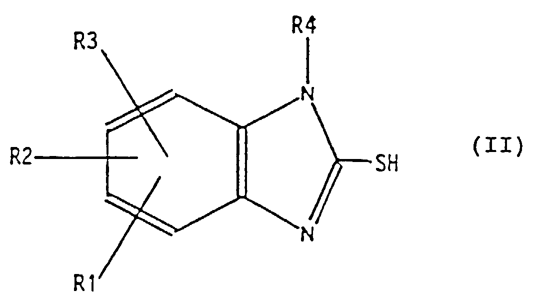 dans laquelle R1, R2, R3 et R4 ont les significations indiquées dans la revendication 1, avec les dérivés de la picoline de formule III : 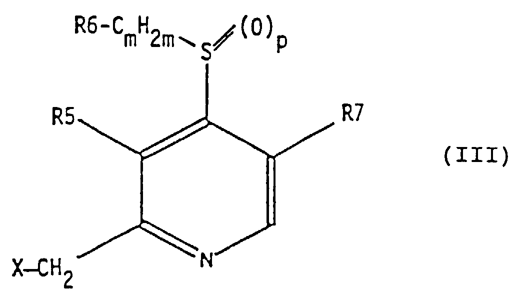 dans laquelle R5, R6, R7, m et p ont les significations indiquées dans la revendication 1 et X représente un groupe labile approprié, et on oxyde ensuite les sulfures de 2-benzimidazolyle et de 2-pyridylméthyle obtenus correspondant à la formule I avec n = 0, dans le cas où les produits finaux désirés sont des composés de formule I avec n = 1, et/ou on les transforme éventuellement en leurs sels.

8. Médicament contenant un ou plusieurs composés de formule I selon la revendication 1 et/ou leurs sels pharmacologiquement admissibles.

9. Composés de formule I selon la revendication 1 et/ou leurs sels pharmacologiquement admissibles pour l'utilisation dans la lutte contre les bactéries Helicobacter.

10. Utilisation des composés de formule I selon la revendication 1 et de leurs sels pharmacologiquement admissibles pour la préparation de médicaments destinés à la lutte contre les bactéries Helicobacter.
